Europäisches Patentamt

European Patent Office

Office européen des brevets

(11) Publication number: **0 340 171**
**A2**

## (12) EUROPEAN PATENT APPLICATION

(21) Application number: **89810298.3**

(22) Date of filing: **21.04.89**

(51) Int. Cl.⁴: **C 08 B 37/16**
**A 61 K 7/22**

(30) Priority: **27.04.88 US 186536**

(43) Date of publication of application:
**02.11.89 Bulletin 89/44**

(84) Designated Contracting States:
**AT BE CH DE ES FR GB GR IT LI LU NL SE**

(71) Applicant: **WARNER-LAMBERT COMPANY**
**201 Tabor Road**
**Morris Plains New Jersey 07950 (US)**

(72) Inventor: **Gallopo, Andrew Robert**
**133 Wessington Avenue**
**Garfield New Jersey 07026 (US)**

(74) Representative: **Silbiger, Jakob, Dr.**
**c/o CAPSUGEL AG Münchensteinerstrasse 41**
**CH-4002 Basel (CH)**

(54) Cyclodextrin complexes of 5-aminohexahydropyrimidine compounds.

㊟ The invention provides cyclodextrin complexes of 5-aminohexahydropyrimidine compounds such as hexetidine and its salts which act to increase the water solubility of the hexetidine or its salts and increase their bioavailability.

The invention also provides aqueous-based antibacterial oral preparations containing such complexes which may also contain colorants, flavorants, sweeteners, fluorides and polishing agents.

EP 0 340 171 A2

Bundesdruckerei Berlin

**Description**

# CYCLODEXTRIN COMPLEXES OF 5-AMINOHEXAHYDROPYRIMIDINE COMPOUNDS

This invention is concerned with cyclodextrin complexes of 5-aminohexahydropyrimidine compounds, such as hexetidine, and an anti-bacterial oral composition containing such cyclodextrin complexes.

5-aminohexahydropyrimidine compounds such as hexetidine are well known in the art for their anti-bacterial activity and have been used in aqueous-based oral compositions to counter dental plaque and caries formation by bacteria in the oral cavity.

Hexetidine -- also known as 5-amino-1,3 bis(2-ethylhexyl)-5-methyl-hexahydropyrimidine)-- is soluble in ethanol but is practically insoluble in water. Therefore, in oral hygiene products containing hexetidine solubility is an important consideration in determining the amount of hexetidine that can be present in the ultimate product. In formulating a composition containing an effective level of hexetidine it is usually necessary to add ethanol to aid in dissolving the hexetidine. This can be disadvantageous because there can be instances when it would be desirable to have minimal or substantially no ethanol present in the product. For example, under normal circumstances a composition containing about 0.1% by weight hexetidine may also contain about 9.5% ethanol. Such a level of ethanol may result in the consumer experiencing an unpleasant burning sensation.

Hexetidine also has the disadvantage that it stains the teeth with repeated use and has a bitter taste.

Thus, it would be commercially advantageous to increase the water solubility of hexetidine so that it can be more readily incorporated into aqueous based anti-bacterial compositions and find greater acceptability to the user. It would also be advantageous if the bioavailability of hexetidine could be increased so that less hexetidine would be needed to accomplish the same result as a larger quantity of hexetidine. Stated another way, it would be advantageous if the bioavailability of a given quantity of hexetidine could be increased. By increasing the bioavailability a final product could contain less hexetidine without a concomitant decrease in therapeutic benefits. This invention provides cyclodextrin complexes of 5-aminohexahydropyrimidine compounds, particularly hexetidine, having one or more of the aforementioned desirable advantages.

Cyclodextrins have long been known to form inclusion complexes with various compounds. The cyclodextrin molecule comprises a plurality of glucopyranose units arranged in a torus-like configuration having all the secondary hydroxy groups located on one side of the torus and all primary hydroxyl groups located on the other side. Alpha, beta, and gamma cyclodextrin contain 6, 7 & 8 cyclic glucopyranose units, respectively, in the torus shell. The "lining" of the internal cavity is formed by hydrogen and glucosidic oxygen-bridge atoms and therefore the surface is slightly apolar.

Molecules, or functional groups of molecules having molecular dimensions that match the cyclodextrin cavity, being less hydrophilic than water, can be included into the cyclodextrin cavity in the presence of water. In aqueous solution the slightly apolar cyclodextrin cavity is occupied by water molecules which are energetically unfavored (polar-apolar interaction) and are therefore readily substituted by appropriate "guest molecules" which are less polar than water.

Almost all industrial applications of cyclodextrins involve complexation and include complexation of flavors, fragrances, drugs and pesticides to protect them against the action of heat, light, oxygen and the loss of volatile constituents.

Heretofore, 5-aminohexahydropyrimidine compounds such as hexetidine have not been complexed with cyclodextrins.

The invention provides cyclodextrin complexes of 5-aminohexahydropyrimidine compounds, particularly hexetidine, which are formed by dissolving at least a stoichiometric amount of cyclodextrin in deionized water at room temperature and adding hexetidine thereto. Preferably a molar excess of cyclodextrin is used, and preferably the hexetidine is added to the cyclodextrin at elevated temperatures. The hexetidine-cyclodextrin complex is isolated by filtering out the precipitate formed, or by cooling, freezing and lyophilizing the solution containing the complex. Beta-cyclodextrin complex formation was confirmed by NMR analysis.

The cyclodextrin complexes act to increase the water solubility of the corresponding hexetidine from about 0 to about 0.1% w/v rendering it more easily incorporated into aqueous-based compositions. The complexes also act to increase hexetidine's bioavailability.

The invention also provides aqueous-based anti-bacterial oral preparations containing these complexes which may also contain flavorants, colorants, sweeteners, fluorides and polishing agents.

The preferred 5-aminohexahydropyrimidines which may be employed in the present invention includes hexetidine and may include salts thereof. For example, U.S. 4,141,968, the disclosure of which is incorporated herein by reference thereto, discloses salts of hexetidine with substituted benzoic acids, particularly terephthalic acid and 4-sulfamoylbenzoic acid. The substituents on the benzoic acid ring may be one or two in number. The substituents may be selected from the group consisting of -COOH,-OH, $NH_2$,-$SO_3H$, and $SO_2NH_2$. When the substituent is -COOH or -OH it is in the para position. When there are two substituents they are each different.

Preferably, there is one substituent in the para position. Examples include:

1,3-bis(beta-ethylhexyl)-5-amino-5-methylhexahydropyrimidineterephthalate;

1,3-bis(beta-ethylhexyl)-5-amino-5-methylhexahydropyrimidine-4-sulfamoylbenzoate;

1,3-bis(beta-ethylhexyl)-5-amino-5-methylhexahydropyrimidine-4-hydroxybenzoate;
1,3-bis(beta-ethylhexyl)-5-amino-5-methylhexahydropyrimidine-2-aminobenzoate;
1,3-bis(beta-ethylhexyl)-5-amino-5-methylhexahydropyrimidine-4-aminobenzoate;
1,3-bis(beta-ethylhexyl)-5-amino-5-methylhexahydropyrimidine-4-aminosalicylate; and
1,3-bis(beta-ethylhexyl)-5-amino-5-methylhexahydropyrimidine-5-sulfosalicylate.

The cyclodextrins, according to the invention, include alpha, beta and gamma cyclodextrin containing 6, 7 and 8 cyclic glucopyranose units, respectively, in the torus shell. Beta-cyclodextrin is preferred due to its availability and efficiency in forming complexes. Betacyclodextrin is also preferred because a cyclodextrinhexetidine complex can yield a solution of 0.1% w/v of hexetidine. In addition, the beta-cyclodextrin complex is preferred because this complex can be dissolved in an aqueous solution without using ethanol or surfactants.

The cyclodextrin complexes according to the invention can be formed by first dissolving cyclodextrin in deionized water. The hexetidine compound can then be added with mixing at room temperature (about 25°C) until the complex is formed. Generally, the cyclodextrin should be present in at least stoichiometric amounts with respect to the hexetidine compound, with a molar excess being preferred and about a 0.25 to about a 0.75 molar excess being most preferred, and about a 0.5 molar excess being most preferred. The ratio of cyclodextrin to hexetidine compound is usually within the range of about 1:1 to about 1.5:1.

Although complex formation does take place at room temperature, the rate of formation is relatively slow; therefore, it is preferred, to speed up the rate of complex formation, to elevate the temperature of the cyclodextrin solution and then add the hexetidine compound. The cyclodextrin solution can be heated to any suitable temperature which allows complex formation to proceed at a reasonable rate but which does not adversely affect the cyclodextrin, hexetidine compound, or the complex which is formed. Generally, the cyclodextrin solution is heated to a temperature of about 50° to about 80°C, with about 65° to about 80°C being preferred and about 70°C being most preferred.

The mixture is then cooled to room temperature. The complex may be present as a precipitate or may remain in solution. The precipitated complex may be isolated by filtration, and the complex remaining in solution may be isolated by freezing and then lyophilizing --i.e. freeze drying-- to a solid. If convenient the complex may be used as it is formed without isolating it.

Evidence of the formation of the beta-complex has been shown by NMR, UV and increased water-solubility of the complex. Evidence of the formation of the gamma complex has been shown by UV and increased water solubility.

It has been shown that by complexing with cyclodextrins, hexetidine exhibits increased solubility. For example, hexetidine is virtually insoluble in water. When complexed with beta-cyclodextrin the solubility increases to about 10 mg hexetidine (contained in about 43 mg of complex) in about 10 ml of water -- without the addition of any alcohol or any surfactant. This is an increase from about 0 to about 0.1% w/v in solubility.

When complexed with gamma-cyclodextrin, the hexetidine has a solubility of about 10 mg (contained in about 48 mg of complex) in a mixture of about 10 ml water and about 0.4 ml ethanol -- i.e., about a 3.8% v/v solution of ethanol provides about a 0.096% w/v solution of hexetidine without the use of a surfactant. This may be compared with known in the art compositions which require a solution of about 9.5% v/v ethanol and about 0.7% v/v of surfactant (e.g., TWEEN 80) to produce about a 0.1% w/v solution of hexetidine.

Similarly, when complexed with gamma-cyclodextrin, hexetidine has a solubility of about 10 mg (contained in about 48 mg of complex) in a mixture of about 15 ml water and about 0.4 ml alcohol -- i.e., about a 2.6% v/v ethanol solution provides about a 0.065% w/v solution of hexetidine without the addition of a surfactant.

Therefore, the beta-cyclodextrin complexes of hexetidine result in the formation of about a 0.1% w/v aqueous solution of hexetidine (no ethanol and no surfactant present), and the gamma cyclodextrin complexes result in the formation of about a 0.07% or about a 0.1% w/v aqueous solution of hexetidine using substantially less ethanol than known in the art compositions without the need for a surfactant. This increased solubility is further evidence of complex formation.

Also, it has been found, that cyclodextrin complexes of hexetidine compounds increase its bioavailability. In tests for bioavailability of hexetidine and its cyclodextrin complex a 0.1% w/v aqueous solution of hexetidine as the complex demonstrated an antimicrobial activity equivalent to a 0.185% w/v solution of hexetidine alone.

The cyclodextrin complexes of the invention can be readily incorporated into aqueous or aqueous-alcohol based oral preparations such as a mouthwash, spray, rinse, toothpaste, dental cream or toothpowder.

The complex is present in amounts sufficient to provide the desired level of hexetidine compound in the oral preparation. These amounts are readily determined by those skilled in the art without the need for undue experimentation. Generally, a mouthwash (or rinse) will contain about 0.025 to about 0.1% w/v hexetidine compound with about 0.1% w/v being preferred; and a spray will contain about 0.05% to about 0.3% w/v hexetidine, with about 0.2% w/v being preferred. Those skilled in the art will appreciate that the maximum amount of a hexetidine compound which can be present in a dentifrice is related to the compound's solubility in the aqueous vehicle used, generally a dentifrice (e.g., toothpaste, dental cream, gel or toothpowder) will contain about 0.1 to about 1% w/w hexetidine with about 0.5% w/w being preferred.

The beta-complex of hexetidine is soluble in aqueous solution without having to use alcohol (ethanol) and surfactants. The gamma-complex of hexetidine is soluble, without having to use surfactants, in aqueous solutions containing smaller amounts of alcohol then known in the art compositions. A sufficient amount of alcohol is used to dissolve the desired amount of gamma complex in the aqueous solution. Generally, for example, amounts of about 5% v/v of ethanol, with about 2% to about 4% v/v being preferred, and about 2.5% to about 4% v/v being most preferred are utilizable to produce an aqueous solution containing from about 0.06% w/v to about 0.1% w/v hexetidine.

If desired, however, typical vehicles known in the art may be used. In such a preparation the vehicle is typically a water-alcohol mixture. Generally the ratio of total water to alcohol is in the range of from about 1:1 to about 20:1, preferably about 3:1 to about 20:1 and most preferably about 3:1 to about 10:1 by weight. The total amount of water-alcohol mixture in a mouthwash preparation is typically in the range from about 45% to about 82.5% by weight of the composition.

The pH value of such mouthwash preparations is generally from about 4 to about 9 and preferably from about 5 to about 7. A pH below 4 is irritating to the oral cavity and a pH greater than 9 results in an unpleasant mouth feel.

Oral liquid preparations may also contain surface active agents --i.e. surfactants-- in amounts up to about 5% and fluorine-providing compounds in amounts up to about 2% by weight of the preparation.

Surface active agents (surfactants) are organic materials which aid in the complete dispersion of the preparation throughout the oral cavity. The organic surface active material may be anionic, non-ionic, ampholytic, or cationic. Suitable anionic surfactants are water-soluble salts of higher fatty acid monoglyceride monosulfates, such as the sodium salt of the monosulfated monoglyceride of hydrogenated coconut oil fatty acids; higher alkyl sulfates, such as sodium lauryl sulfate; alkyl aryl sulfonates, such as sodium dodecyl benzene sulfonate; higher alkyl sulfonacetates; higher fatty acid esters of 1,2-dihydroxy propane sulfonates; and substantially saturated higher aliphatic acyl amides of lower aliphatic amino carboxylic acids such as those having 12 to 16 carbons at the fatty acid, alkyl or acyl radicals. Examples of the last mentioned amides are N-lauroyl sarcosine, and the sodium, potassium, and ethanolamide salts of N-lauroyl, N-myristyl or N-palmitoyl sarcosine.

The non-ionic surfactants employed are poly(oxyethylene)-poly(oxypropylene) block copolymers. Such copolymers are known commercially as poloxamers and are produced in a wide range of structures and molecular weights with varying contents of ethylene oxide and propylene oxide. The non-ionic poloxamers according to the invention are non-toxic and acceptable as direct food additives. They are stable and readily dispersible in aqueous systems and are compatible with a wide variety of formulating ingredients for oral preparations. These surfactants should have an HLB (Hydrophilic-Lipophilic Balance) of between about 10 and 30 and preferably between 10 and 25.

Thus, non-ionic surfactants useful in this invention include poloxamers:

| 105 | 188 | 284 |
| 108 | 215 | 288 |
| 123 | 217 | 334 |
| 124 | 234 | 335 |
| 183 | 235 | 338 |
| 184 | 237 | 407 |
| 185 | 238 | |

Generally these polymers should constitute from 0.2% to 2% by weight of total volume of liquid oral preparation (% w/v) and preferably from 0.5% to 1% w/v. A particularly preferred poloxamer is Poloxamer 407 having an HLB of about 22. Such a polymer is sold under the trademark Pluronic F-127 (BASF-WYANDOTTE). Another class of non-ionic surfactants useful in this invention are ethoxylated hydrogenated castor oils. Such surfactants are prepared by hydrogenating castor oil and treating the so-formed product with from about 10 to 200 moles of ethylene glycol. They are designated as PEG (numeral) hydrogenated castor oil in accordance with the dictionary of the Cosmetics, Toiletries and Fragrance Association, 3rd Ed. wherein the numeral following PEG indicates the degree of ethoxylation, i.e. the number of moles of ethylene oxide added. Suitable PEG hydrogenated castor oils include PEG 16, 20, 25, 30, 40, 50, 60, 80, 100 and 200. The ethoxylated hydrogenated castor oils are used in the same concentrations as the above described poly(oxyethylene)-poly(oxypropylene) block copolymers.

Other non-ionic surface active agents which may be suitable include codensates of sorbitan esters of fatty acids with from 20 to 60 moles of ethylene oxide (e.g., "Tweens" a trademark of ICI United States, Inc.), and amphoteric agents such as quaternized imidazole derivatives.

Additional non-ionic surfactants which may be suitable are the condensation products of an alpha-olefin oxide containing 10 to 20 carbon atoms, a polyhydric alcohol containing 2 to 10 carbons and 2 to 6 hydroxyl groups and either ethylene oxide or a mixture of ethylene oxide and propylene oxide. The resultant surfactants are polymers having a molecular weight in the range of 400 to about 1600 and containing 40% to 80% by weight of ethylene oxide, with an alpha-olefin oxide to polyhydric alcohol mole ratio in the range of about 1:1 to 1:3.

Cationic surface active agents which may be suitable are molecules that carry a positive charge such as cetylpyridinium chloride.

Fluorine providing compounds may be present in the oral preparations of this invention. These compounds may be slightly water soluble or may be fully water soluble and are characterized by their ability to release fluoride ions or fluoride containing ions in water. Typical fluorine providing compounds are inorganic fluoride salts such as soluble alkali metal, alkaline earth metal, and heavy metal salts, for example, sodium fluoride, potassium fluoride, ammonium fluoride, cuprous fluoride, zinc fluoride, stannic fluoride, stannous fluoride, barium fluoride, sodium fluorosilicate, ammonium fluorosilicate, sodium fluorozirconate, sodium monofluorophosphate, aluminum mono- and difluorophosphate and fluorinated sodium calcium pyrophosphate.

Alkali metal, tin fluoride and monofluorophosphates such as sodium and stannous fluoride, sodium monofluorophosphate and mixtures thereof are preferred.

In an oral liquid preparation such as a mouthwash, the fluorine providing compound is generally present in an amount sufficient to release up to about 0.15%, preferably about 0.001% to about 0.1% and most preferably from about 0.001% to about 0.05% fluoride by weight of the preparation.

The oral preparation may also contain additional flavorants and colorants.

In the instance where auxiliary sweeteners are utilized, the present invention contemplates the inclusion of those sweeteners well known in the art, including both natural and artificial sweeteners. Thus, additional sweeteners may be chosen in minor amounts from the following non limiting list provided they do not inactivate the hexetidine compound.

The sweetening agent (sweetener) used may be selected from a wide range of materials including water-soluble sweetening agents, water-soluble artificial sweeteners, water-soluble sweetening agents derived from naturally occurring water-soluble sweeteners, dipeptide based sweeteners, and protein based sweeteners, including mixtures thereof. Without being limited to particular sweeteners, representative illustrations encompass:

A. Water-soluble sweetening agents such as monosaccharides, disaccharides and polysaccharides such as xylose, ribose, glucose (dextrose), mannose, galactose, fructose (levulose), sucrose (sugar), maltose, invert sugar (a mixture of fructose and glucose derived from sucrose), partially hydrolyzed starch, corn syrup solids, dihydrochalcones, monellin, steviosides, and glycyrrhizin;

B. Water-soluble artificial sweeteners such as the soluble saccharin salts, i.e. sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (acesulfame-K), the free acid form of saccharin, and the like;

C. Dipeptide based sweeteners, such as L-aspartic acid derived sweeteners, such as L-aspartyl-L-phenylalanine methyl ester (aspartame) and materials described in U.S. Pat. No. 3,492,131, L-α-aspartyl-N-(2,2,4,4-tetramethyl-3-thietanyl)-D-alaninamide hydrate, methyl esters of L-aspartyl-L-phenylglycerine and L-aspartyl-L-2,5,dihydrophenylglycine, L-aspartyl-2,5-dihydro-L-phenylalanine; L-aspartyl-L-(1-cyclohexyen)-alanine; and the like;

D. Water-soluble sweeteners derived from naturally occurring water-soluble sweeteners, such as a chlorinated derivative of ordinary sugar (sucrose), known, for example, under the product description of sucralose; and

E. Protein based sweeteners such as thaumatoccous danielli (Thaumatin I and II).

In general, an effective amount of auxiliary sweetener is utilized to provide the level of sweetness desired for a particular composition, and this amount will vary with the sweetener selected. This amount will normally be 0.01% to about 40% by weight of the composition when using an easily extractable sweetener. The water-soluble sweeteners described in category A above, are usually used in amounts of about 5% to about 40% by weight, and preferably in amounts of about 10% to about 20% by weight of the final composition. Some of the sweeteners in category A (e.g., glycyrrhizin) may be used in amounts set forth for categories B-E below due to the sweeteners' known sweetening ability. In contrast, the sweeteners described in categories B-E are generally used in amounts of about 0.005% to about 5.0% by weight of the final composition with about 0.03% to about 2.5% by weight being usual and about 0.03 to about 0.4% by weight being preferred. These amounts may be used to achieve a desired level of sweetness independent from the flavor level achieved from any optional flavor oils used.

Suitable flavorings include both natural and artificial flavors, and mints such as peppermint and spearmint. Citrus flavors such as orange and lemon, various fruit flavors, both individual and mixed, and the like are contemplated. Aldehyde-containing flavors are to be avoided as aldehydes generally react with hexetidine to form Schiff bases. The flavorings are generally utilized in amounts that will vary depending upon the individual flavor, and may, for example, range in amounts of about 0.05% to about 6% by weight of the final composition.

The colorants useful in the present invention include the pigments which may be incorporated in amounts of up to about 2% by weight of the composition. Also, the colorants may include other dyes suitable for food, drug and cosmetic applications, known as FD & C and D & C dyes. The materials acceptable for the foregoing spectrum of use are preferably water-soluble. Illustrative examples include the yellow dye, known as D & C Yellow # 10, and the dye known as FD & C Green # 3 which comprises a triphenylmethane dye. A full recitation of all FD & C and D & C colorants useful in the present invention and their corresponding chemical structures may be found in the Kirk-Othmer Encyclopedia of Chemical Technology, 3rd Edition, in Volume 6, at pages 561-595, which text is accordingly incorporated herein by reference.

The oral preparations may also be substantially solid or pasty in character such as a dental cream, toothpaste or a toothpowder. Solid or pasty oral preparations contain polishing materials. Typical polishing materials are abrasive particulate materials having particle sizes of up to about 20 microns. Nonlimiting illustrative examples include: water-insoluble sodium metaphosphate, potassium metaphosphate, tricalcium phosphate, dihydrated calcium phosphate, calcium pyrophosphate, magnesium orthophosphate, trimagnesium phosphate, calcium carbonate, alumina, aluminum silicate, zirconium silicates, silica, bentonite, and mixtures thereof. Polishing materials are generally present in an amount from about 20% to about 82% by weight of the oral preparation. Preferably, they are present in amounts from about 20% to about 75% in toothpaste, and from about 70% to about 82% in toothpowder. For toothpaste and dental creams the water content is about 25% to 50% by weight.

In clear gels, a polishing agent of colloidal silica and alkali metal aluminosilicate complexes are preferred since they have refractive indicies close to the refractive indicies of gelling agent liquid systems commonly used in dentrifices.

In the oral preparations that are toothpastes, dental creams, or gels the liquid vehicle may comprise water, typically in an amount of about 10-90% by weight of the composition. Polyethylene glycol, propylene glycol, glycerin or mixtures thereof may also be present as humectants or binders in amounts of about 20-25% by weight. Particularly advantageous liquid ingredients comprise mixtures of water with polyethylene glycol or glycerin and propylene glycol. A gelling agent (thickening agent) including natural or synthetic gums such as sodium carboxymethylcellulose, hydroxyethyl cellulose, methyl cellulose and the like may be used, in the range of about 0.5-5% by weight. The preferred gelling agent is hydroxyethyl cellulose. In a toothpaste, dental cream or gel, the liquids and solids are proportioned to form a creamy or gelled mass which is extrudable from a pressurized container or from a collapsible tube.

The toothpaste or gel may also contain a surface active agent which may be an anionic, nonionic or zwitterionic detergent (surfactant) in amounts of about 0.05-5% by weight. The anionic and nonionic surfactants that are suitable have already been discussed above.

Zwitterionic surface active agents include the betaines and sulfobetaines. Typical alkyl dimethyl betaines include decyl betaine or 2-(N-decyl-N,N-dimethylammonio)acetate, coco betaine, myristyl betaine, palmityl betaine, lauryl betaine, cetyl betaine, stearyl betaine, etc. The amidobetaine similarly include cocoamidoethyl betaine, cocoamidopropyl betaine, lauramidopropyl betaine and the like. These sulfobetaines are similar in structure to the betaines, but have a sulfonate group in place of the carboxylate group, and include alkysulfobetaines, alkylamidosulfobetaines and alkylaminosulfobetaines.

In general, the anti-bacterial oral compositions of the present invention are prepared as follows. The sweetener is dissolved in water to form a solution. The cyclodextrin complex is added to the solution and mixed until dissolved. Then sufficient water, alcohol or mixtures thereof are added with mixing until the final solution volume is reached. When colorants, additional sweeteners and similar additives are included in the composition, they are added at the same time the sweetener is added. In a preferred embodiment the complex is added as the final ingredient.

Those skilled in the art will appreciate that the total amount of all the components of a composition equals 100%.

The following examples are illustrative only and should not be construed as limiting the invention in any way. Those skilled in the art will appreciate that variations are possible which are within the spirit and scope of the appended claims.

### EXAMPLE 1

A hexetidine-beta-cyclodextrin complex was prepared as follows.

Beta cyclodextrin (M.W. 1135), 1.70 g (1.5 millimoles), was dissolved in 100 ml of deionized water in a flask, hexetidine (M.W. 340, density 0.8889 g/ml), 0.38 ml (1.0 millimole) was added to the beta-cyclodextrin solution. The resulting mixture was heated to 70°C with stirring. This mixture was allowed to stir and cool down slowly -- i.e., over about a 3 to 4 hour time period. Stirring was then discontinued and a solid material settled to the bottom of the flask. The solid material (hexetidine-beta-cyclodextrin complex) was separated from the solution by filtration and then air dried to produce 1.35g of a white dried material.

The solid had no odor and had a bitter taste. Complex formation was confirmed by NMR analysis of the solid material.

The complex, 43 mg, was dissolved in 10 ml of deionized water at about 50°C using vortexing. The complex, assayed by UV to be a 1:1 complex of hexetidine: beta-cyclodextrin, contains 10 mg of hexetidine in the 43 mg of complex, and therefore, a 0.1% w/v solution of hexetidine was made. The solution of the 43 mg of complex in 10 ml of water was clear. In comparison, when it was attempted to dissolve 10 mg of hexetidine alone in 10 ml of water, the hexetidine did not dissolve, as indicated by the cloudy solution obtained.

### EXAMPLE 2

A hexetidine-gamma-cyclodextrin complex was prepared in accordance with the procedure of Example 1, except as follows.

Gamma-cyclodextrin (M.W. 1297), 973 mg (0.75 millimole) was dissolved in 50 ml of deionized water. Hexetidine, 0.19 ml (0.5 millimole, 170 mg) was then added to the gamma cyclodextrin. A white solid material, 865 mg, was obtained.

The complex, 48 mg, was dissolved in a mixture of 10 ml deionized water and 0.4 ml ethanol. The complex, a 1:1 complex of hexetidine:gamma-cyclodextrin (based on the 1:1 hexetidine: beta-cyclodextrin complex shown by UV analysis to have been formed in Example 1), contains 10 mg of hexetidine in the 48 mg of complex; and therefore, a 0.096% w/v hexetidine solution containing 3.8% v/v ethanol was made.

Similarly, 48 mg of complex was dissolved in a mixture of 15 ml of deionized water and 0.4 ml of ethanol to produce a 0.065% w/v hexetidine solution containing 2.6% ethanol.

The complex formation is demonstrated by the increased solubility and by UV spectra.

## EXAMPLE 3

A hexetidine-alpha-cyclodextrin complex was prepared in accordance with the procedure of Example 1, except as follows.

Alpha-cyclodextrin (M.W. 973) 1.46g (1.5 millimoles), was dissolved in 100ml of deionized water. Hexetidine, 0.38ml (1.0 millimole) was then added to the alphacyclodextrin. The resulting solution was stirred for about 48 hours after which time the solution was still cloudy. The solution was then heated to about 65°-70°C for about three hours with continued stirring. After this time period the hexetidine-alpha-cyclodextrin complex was believed to have been formed. The solution was freeze dried, using known in the art procedures, which resulted in the isolation of a white dried solid.

## EXAMPLE 4

Using another test, an agar diffusion assay, it was found that an aqueous solution of the beta-cyclodextrin complex of hexetidine having a theoretical hexetidine level in the complex of 0.1% by weight, based on the weight of the complex, demonstrated anti-microbial activity equivalent to a 0.185% by weight solution of hexetidine alone. This result shows an increased bioavailability of the hexetidine possibly due to increased solubility in the agar medium.

The invention being thus described, it will be obvious that the same may be varied in many ways. Such variations are not to be regarded as a departure from the spirit and scope of the invention and all such modifications are intended to be included within the scope of the claims.

## Claims

1. A composition of matter comprising a cyclodextrin complex of a 5-aminohexahydropyrimidine compound.

2. The composition of Claim 1 wherein said 5-aminohexahydropyrimidine compound is selected from the group consisting of hexetidine, salts of hexetidine, and mixtures thereof.

3. The composition of Claim 1 or 2 wherein said cyclodextrin is selected from the group consisting of alpha-cyclodextrin, beta-cyclodextrin and gamma-cyclodextrin.

4. A composition of matter comprising an alpha-cyclodextrin complex of hexetidine.

5. A composition of matter comprising a beta-cyclodextrin complex of hexetidine.

6. A composition of matter comprising a gamma-cyclodextrin complex of hexetidine.

7. A method of preparing a cyclodextrin complex of a hexetidine compound comprising:

(a) forming an aqueous solution of cyclodextrin;

(b) adding said hexetidine compound to said solution; and

(c) mixing the resulting solution.

8. The method of Claim 7 which further comprises heating said solution of cyclodextrin to an elevated temperature.

9. The method of Claim 7 which further comprises filtering the resulting solution to recover said complex.

10. The method of Claim 8 which further comprises cooling the resulting solution.

11. The method of Claim 10 which further comprises freezing said resulting solution.

12. The method of Claim 11 which further comprises lyophilizing said resulting solution to recover said complex.

13. The method of Claim 8 wherein said elevated temperature is between about 65° and about 80°C and preferably is about 70°C.

14. The method of Claim 7 wherein said cyclodextrin is present in said solution in a molar excess of from about 0.25 to about 0.75 of said hexetidine compound.

15. A composition of matter according to anyone of the Claims 1 to 6 being an aqueous based oral preparation.

16. The oral preparation of Claim 15 wherein said oral preparation is selected from the group consisting of mouthwashes, sprays, and dentifrices; and wherein said 5-aminohexahydropyrimidine compound is present in amounts of about 0.025 to about 0.1% w/v in said mouthwash, in amounts of about 0.05% to about 0.3% w/v in said spray, and in amounts of about 0.1% to about 1% w/v in said dentifrice.

17. The oral preparation of Claim 15 or 16 wherein the oral preparation is a mouthwash and the complex is a beta-cyclodextrin complex of hexetidine.

18. The oral preparation according to anyone of the Claims 15 to 17 which further comprises about 5% w/v of ethanol.

19. The oral preparation according to anyone of the Claims 15 to 18 which further comprises an additive selected from the group consisting of a fluorine providing compound, a colorant, a flavorant, an artificial sweetener, a natural sweetener, a polishing agent and mixtures thereof.